# EUROPEAN PATENT APPLICATION

(11) **EP 4 006 625 A1**
(43) Date of publication of application: **01.06.2022**
(21) Application number: 20843161.9
(22) Date of filing: 10.07.2020
(51) Int. Cl.: G02C 7/04, C08L 33/06, B29D 11/00, B29C 33/00, B29C 33/38

(54) **CONTACT LENS AND MOLD FOR MANUFACTURING SAME**

(30) Priority: 24.07.2019 KR 20190089831
(71) Applicant: Dae Won Pharmaceutical Co., Ltd., Seoul 04808 (KR)
(72) Inventor: JEONG, Ok Chan, Busan 46539 (KR); CHOI, Du Hyung, Daegu 42278 (KR); LEE, Ah-Ram, Incheon 22003 (KR); PARK, Sang-Wook, Seoul 04572 (KR)
(74) Representative: Lavoix
(86) International application number: PCT/KR2020/009084
(87) International publication number: WO 2021/015465

(57) **Abstract**

According to an embodiment, a contact lens includes: a vision correction lens portion positioned in a center portion of the contact lens to refract light; and a plurality of tear storage structures radially arranged from the center portion of the contact lens to store tears, wherein each of the plurality of tear storage structures includes a concave structure formed in the contact lens and having a first depth to store introduced tears.

According to an embodiment, a contact lens includes: a vision correction lens portion positioned in a center portion of the contact lens to refract light; and a drug storage structure arranged apart from the center portion and configured to store a drug, wherein the drug is provided to an eye of a user wearing the contact lens.

## Description

### TECHNICAL FIELD

The present disclosure relates to a contact lens and a mold for manufacturing the contact lens.

### BACKGROUND ART

Contact lenses refract light incident on the eyes to correct vision, and unlike glasses, contact lenses are worn on the front surfaces of the eyes, mainly on the corneas. Most contact lenses correct refractive errors such as nearsightedness, astigmatism, and farsightedness, and are thus used for correcting vision. Contact lenses are commonly used by people who do not want to wear glasses for cosmetic purposes. Moreover, when sufficient vision correction is not obtained with glasses in cases such as severe nearsightedness, severe astigmatism, anisometropia (large difference in the refractive power of the two eyes: commonly called "unbalanced eyesight"), severe farsightedness (especially when the eye lens is removed through surgery or due to injury), irregular astigmatism (when the corneal surface is irregular), keratoconus (when the corneal surface protrudes), contact lenses may provide better vision improvement than glasses.

Contact lenses are worn on the corneas with secreted tears therebetween. When tears are normally secreted, there is no problem, but it is difficult for people with dry eye syndrome (dry eyes) to wear contact lenses due to a severe foreign body sensation.

### DESCRIPTION OF EMBODIMENTS

### TECHNICAL PROBLEM

Dry eye syndrome refers to the condition in which the surface of the eye is dry and lacks lubrication by a film of tears because of insufficient tears, an insufficient component of tears, or excessive evaporation of tears. Therefore, contact lenses cannot be worn due to severe irritation to the eyes.

Furthermore, medicine is administered to patients with various diseases including eye diseases generally by applying eye drops to the eyeball. However, eye drops must be periodically administered, and the effect of eye drops reduces because administered eye drops are discharged together with tears.

Embodiments are provided to address these problems. One objective of embodiments is to provide a contact lens having a tear storage structure on a surface to be brought into contact with the eye for improving dry eye syndrome, and another objective of embodiments is to provide a contact lens capable of continuously supply medicine to a user.

### SOLUTION TO PROBLEM

According to an embodiment, a contact lens includes: a vision correction lens portion positioned in a center portion of the contact lens to refract light; and a plurality of tear storage structures radially arranged from the center portion of the contact lens to store tears, wherein each of the plurality of tear storage structures includes a concave structure formed in the contact lens and having a first depth to store introduced tears.

According to an embodiment, a contact lens includes: a vision correction lens portion positioned in a center portion of the contact lens to refract light; and a drug storage structure arranged apart from the center portion and configured to store a drug, wherein the drug is provided to an eye of a user wearing the contact lens.

According to an embodiment, a mold for forming a contact lens includes: a first mold; and a second mold configured to cure a liquid porous polymer such that the contact lens has a concave structure with respect to the first mold, wherein surfaces of the first mold and the second mold, which are to come into contact with the liquid porous polymer, are coated with Teflon.

### ADVANTAGEOUS EFFECTS OF DISCLOSURE

Embodiments provide contact lenses which patients with dry eye syndrome can wear without irritation to the eyes. Furthermore, embodiments provide contact lenses capable of providing medicine to the eyes of a user. In addition, embodiments provide molds with which contact lenses can be more economically manufactured.

### BRIEF DESCRIPTION OF DRAWINGS

FIG. 1 is a plan view illustrating a contact lens according to an embodiment.
FIG. 2 is a cross-sectional view taken along line A-A' of FIG. 1 to schematically illustrate a tear storage structure.
FIG. 3 is a plan view schematically illustrating an implementation example of a contact lens according to a second embodiment.
FIG. 4 is a cross-sectional view taken along line A-A' of FIG. 3.
FIG. 5 is a cross-sectional view schematically illustrating another implementation example of a contact lens according to a second embodiment.
FIG. 6 is a plan view schematically illustrating another implementation example of the contact lens according to the second embodiment.
FIG. 7 is a schematic cross-sectional view taken along line A-A' of FIG. 6.
FIG. 8A is a cross-sectional view of a contact lens, illustrating examples of a drug outlet 148 in direction B in FIG. 6, and FIG. 8B is a view illustrating another example of the drug outlet 148.
FIG. 9 is a plan view schematically illustrating another implementation example of the contact lens according to the second embodiment.
FIGS. 10A and 10B are views illustrating a mold for manufacturing a contact lens according to an embodiment.
FIG. 11 is an enlarged image taken by a three-dimensional digital microscope (HI-ROX, KH-7700) to illustrate the structure of an implementation example of a contact lens and measure structural dimensions.
FIG. 12 is an image illustrating a manufactured lens mounted on the pupil of an artificial eyeball to test the tear storage effect of the manufactured lens.
FIG. 13 is an image illustrating fluid stored in a tear storage structure as a result of a tear storage experiment performed using the artificial eyeball.
FIG. 14 is a view illustrating drug release patterns for contents of chitosan.
FIG. 15 is a view illustrating drug release patterns in an experiment, for ratios of HPMC 100000 1 % + chitosan 0.2 %.

### MODE OF DISCLOSURE

### First embodiment

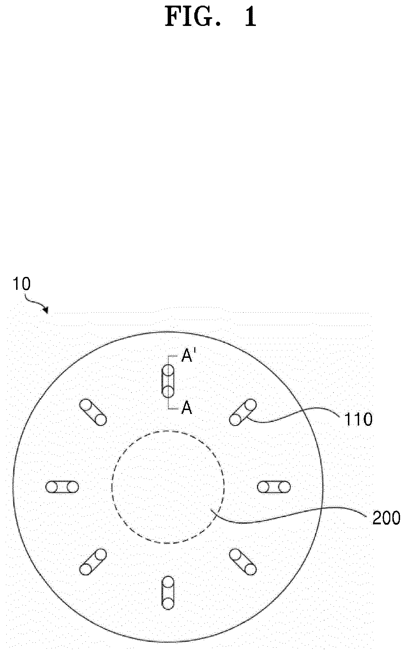

Hereinafter, a contact lens will be described according to a first embodiment with reference to the accompanying drawings. FIG. 1 is a plan view illustrating a contact lens according to an embodiment. Referring to FIG. 1, the contact lens according to the embodiment includes: a vision correction lens portion that is located at the center of the contact lens and configured to refract light for vision correction; and a plurality of tear storage structures that are radially arranged from the center of the contact lens to store tears, wherein each of the tear storage structures includes a concave structure formed in the contact lens a first depth to store introduced tears.

As illustrated in FIG. 1, a plurality of tear storage structures 110 may be radially arranged from the center of a contact lens 10. The contact lens 10 may include eight tear storage structures 110 as shown FIG. 1, but may alternatively include fewer or more tear storage structures.

The contact lens 10 may include a microporous polymer. Therefore, tears stored in the tear storage structures 110 through pores may be provided to the eyeball of a user or may be diffused into the contact lens 10. Thus, owing to the tears provided to the user's eyeball and diffused into the contact lens 10, the user may have a less foreign body sensation with respect to the contact lens 10. In an embodiment, the microporous polymer may be 2-hydroxyethylmethacrylate (2-HEMA), glycerol methacrylate, silicone hydrogel, phosphorylcholine, or the like. In a non-limiting specific example, the microporous polymer may be prepared by mixing 98% or more of 2-HEMA with less than 2% of a crosslinking agent (EGDMA, ethylene glycol dimethacrylate), an initiator (AIBN, 2,2'-azobis(2-methylpropionitrile)), and an organic compound (MAA, methacrylic acid).

The contact lens includes a vision correction lens portion 200 in a center portion thereof, and the vision correction lens portion 200 may be positioned on the cornea to refract light incident to the pupil. As described later, the diopter of the vision correction lens portion 200 may be adjusted by a first mold and/or a second mold, and the vision correction lens portion 200 may be manufactured to have a predetermined diopter for correcting nearsightedness, farsightedness, astigmatism, etc. In an embodiment, the tear storage structures 110 may be radially arranged outward from the vision correction lens portion 200.

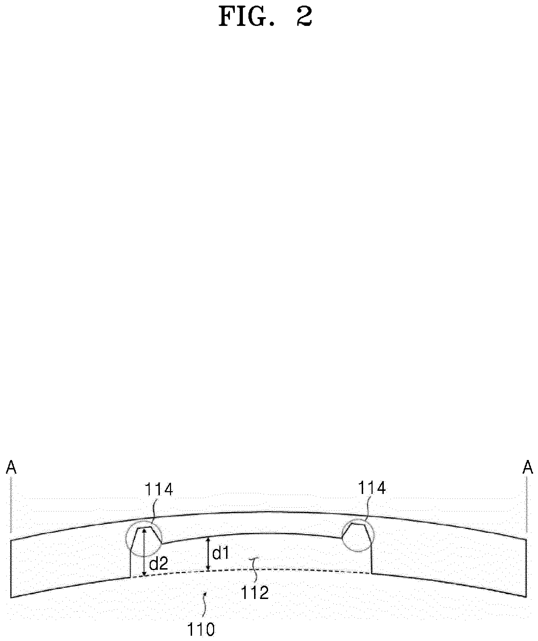

FIG. 2 is a cross-sectional view taken along line A-A' in FIG. 1 to schematically illustrate the tear storage structures 110. Referring to FIG. 2, each of the tear storage structures 110 includes a concave structure 112 formed in the contact lens to a first depth d1 to store introduced tears. The tear storage structure 110 may further include gas discharge structures 114 having a second depth d2 to discharge air remaining in the concave structure 112 to the outside of the contact lens 10.

In FIG. 2, a broken line refers to a contact surface between the contact lens 10 and the eyeball of a user. In general, the eyeball secretes an average of about 0.6 ml of tears every day to prevent the eyeball from drying out. Secreted tears are stored in the concave structure 112 of the contact lens 10 through the contact surface with the user's eyeball. Owing to the concave structure 112, the contact area between the eyeball and the contact lens 10 may be reduced, and the tears stored in the concave structure 112 may be discharged to the user's eyeball or may be diffused into the contact lens 10. Owing to this, the user may have a less foreign body sensation with respect to the contact lens 10.

In an embodiment, the tear storage structure 110 may further include the gas discharge structures 114. In the concave structure 112, gas such as air may be collected together with tears. The gas discharge structure 114 may have the second depth d2 greater than the depth d1 of the concave structure 112 to discharge gas to the outside. As described above, the contact lens 10 includes a porous polymer, and gas collected in the concave structure 112 may have a second depth and may be discharged to the outside through micropores formed in the porous polymer.

In an embodiment, each of the tear storage structures 110 may store 0.1875 mm³ of tears, and when the contact lens 10 has eight tear storage structures 110 as in the embodiment illustrated in FIG. 1, the contact lens 10 may store about 1.5 mm³ or less of tears. In this structure, tears stored in each of the concave structures 112 is discharged to the cornea, and thus when a patient with dry eye syndrome wears the contact lens, the patient may have merits such as minimal evaporation of tears owing to storage of tears and a reduced contact area, and improvements in wearing sensation and minimalization of a foreign body sensation owing to improved lubrication by a film of tears.

### Second embodiment

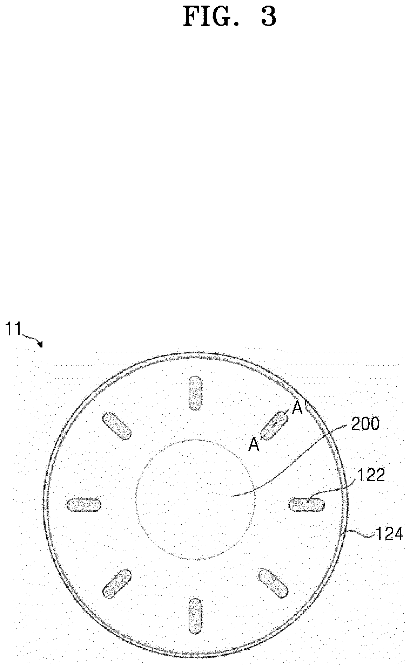

Hereinafter, contact lenses will be described according to a second embodiment with reference to FIGS. 3 to 9. However, descriptions of elements that are the same as or similar to those described above may be omitted. According to the present embodiment, a contact lens may include: a vision correction lens portion that is located in a center portion of the contact lens to refract light; and a drug storage structure that is apart from the center portion and stores a drug, wherein the drug may be provided to the eye of a user of the contact lens.

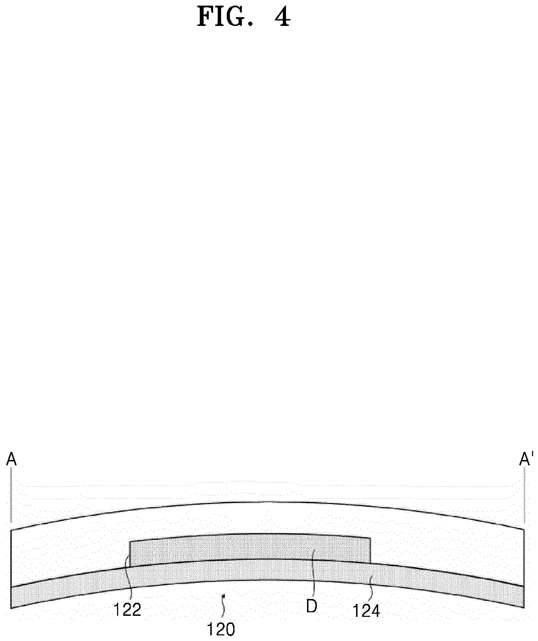

FIG. 3 is a plan view schematically illustrating an implementation example of a contact lens 11 according to the second embodiment, FIG. 4 is a cross-sectional view taken along line A-A' of FIG. 3. Referring to FIGS. 3 and 4, the contact lens 11 includes a drug storage structure 120. The drug storage structure 120 includes: a drug chamber 122 having a concave structure in the contact lens 11 to store a drug D in the concave structure; and a cover member 124 covering at least the drug chamber 122, wherein the cover member 124 includes a microporous polymer in which micropores are formed, and the drug D is provided to the eyeball through the micropores.

The drug chamber 122 may have a concave structure in the contact lens 11. In an embodiment, a plurality of drug chambers 122 may be formed in the single contact lens 11, and the plurality of drug chambers 122 may store different drugs. In another embodiment, the plurality of drug chambers 122 may store the same drug.

The drug chamber 122 may have a predetermined volume. For example, the drug chamber 122 may be configured to store a drug in a volume of 1 µl to 2 µl. In another example, the drug chamber 122 may have a volume corresponding to a single dose of a stored drug. In an embodiment, the drug chamber 122 may store a drug and tears. For example, the drug chamber 122 may be filled with a predetermined amount (eg, 50%) of a drug, and tears may be introduced into the remaining space of the drug chamber 122 and stored in the drug chamber 122. Therefore, the drug may be smoothly released according to the amount of the introduced tears.

The cover member 124 covers the drug chamber 122. In an embodiment, the cover member 124 may cover a rear surface of the contact lens 11 as illustrated in FIG. 3. In an embodiment which is not illustrated, the cover member 124 may cover only the drug chamber 122 such that the stored drug D may not leak.

The cover member 124 may include a microporous polymer in which micropores are formed, and the drug stored in the drug chamber 122 is provided to the eye of a user through the micropores of the cover member 124. The rate and pattern of drug release may be controlled depending on the combination, types, and contents of polymers of the cover member 124.

For example, the cover member 124 may include a water-soluble polymer or a water-insoluble (permeable) polymer. In particular, because the water-insoluble polymer does not dissolve in tears, there is an advantage that a foreign body sensation does not occur in the eye.

In an embodiment, the water-soluble polymer that may be used to form the cover member 124 may include at least one selected from the group consisting of acacia, agar, alginic acid, carbomer, carrageenan, cellulose acetate, ceratonia, chitosan, chondroitin sulfate, dermatan sulfate, dextran, ethyl cellulose, gelatin, guar gum, hydroxyethyl cellulose, hydropropyl betadex, hydroxypropyl cellulose, hypromellose, hypromellose acetate succinate, hypromellose phthalate, karaya gum, locust bean gum, methylcellulose, molasses, pectin, polyacrylamide, polycaprolactone, polyethylene oxide, polyethylene glycol, polyhydroxyethylmethacrylate, polyorthoester, polyvinyl alcohol, polyvinylpyrrolidone, sodium alginate, carboxymethylcellulose, sodium hyaluronate, tragacanth, triethyl citrate, and xanthan gum. However, the scope of the present disclosure is not limited thereto.

In an embodiment, the water-insoluble polymer, which may be used to form the cover member 124, may include at least one selected from the group consisting of acetyl alcohol, acetyl ester wax, acetyl tributyl citrate, aluminum monostearate, carnauba wax, cellulose acetate, cellulose acetate phthalate, dibutyl sebacate, ethyl cellulose, glycerin monostearate, glyceryl behenate, glyceryl monooleate, glyceryl palmitostearate, hydrogenated castor oil, hydrogenated vegetable oil type 1, isopropyl palmitate, polycaprolactone, polyglycolide, polylactic acid, polylactide, polymethacrylate, polyoxyglyceride, shellac, stearic acid, stearyl alcohol, tributyl citrate, white wax, yellow wax, and zein. However, the scope of the present disclosure is not limited thereto.

For example, the drug D, which may be stored in the drug chamber 122 of the contact lens 11 according to the present embodiment, may include at least one selected from the group consisting of a dry eye syndrome treatment drug, a glaucoma treatment drug, an intraocular pressure lowering agent, an eye damage treatment drug, an antibacterial agent, an allergic conjunctivitis treatment drug, a blepharoconjunctivitis treatment drug, a night blindness treatment drug, an amblyopia treatment drug, an eye inflammation treatment drug, a cataract treatment drug, an antiviral agent, a mydriatic drug, a carbonic anhydrase inhibitor, and a macular degeneration treatment drug. However, the scope of the present disclosure is not limited thereto.

In an embodiment, the dry eye syndrome treatment drug may include at least one selected from the group consisting of hyaluronic acid, carboxymethylcellulose, potassium chloride, sodium chloride, hydroxypropyl methylcellulose, chondroitin, glucose, polyvinylpyrrolidone, carbomer, lanolin, dextran, trehalose, sorbic acid, hydroxyethyl cellulose, taurine, polysorbate 80, polyvinyl alcohol, benzalkonium chloride, propylene glycol, macrogol, guar gum, glycerol, cetrimide, glycine, asparagine, retinol palmitate, vitamin E, edetic acid, hydroxymethyl cellulose, phosphoric acid, cyclosporine, diquafosol, povidone, and lipitegrast. However, the scope of the present disclosure is not limited thereto.

In an embodiment, one or more of the glaucoma treatment drug and the intraocular pressure lowering agent may include at least one selected from the group consisting of timolol, dorzolamide, latanoprost, brimonidine, tafluprost, brinzolamide, travoprost, bimatoprost, betaxolol, carteolol, carbachol, nipradilol, apraclonidine, pilocarpine, levobunolol, isopropyl unoprostone, acetylcholine, benzalkonium chloride, befunolol, acetazolamide, methazolamide, diclofenamide, and unoprostone. However, the scope of the present disclosure is not limited thereto.

In an embodiment, the eye damage treatment drug may include at least one selected from the group consisting of ranibizumab, aflibercept, and verteporfin. However, the scope of the present disclosure is not limited thereto.

In an embodiment, the antibacterial agent may include at least one selected from the group consisting of levofloxacin, ofloxacin, tobramycin, moxifloxacin, gatifloxacin, oxytetracycline, polymyxin B, sulfamethoxazole, taurine, glycyrrhizic acid, tosufloxacin, aminocaproic acid, lomefloxacin, chloramphenicol, dexamethasone, tetrahydrozoline, chlorpheniramine, natamycin, ciprofloxacin, enoxolone, fusidic acid, guaiazulene, azulene, erythromycin, colistin, gentamicin, benzalkonium chloride, sulfamethizol, cefmenoxime, norfloxacin, micronomycin, and tetracycline. However, the scope of the present disclosure is not limited thereto.

In an embodiment, one or more of the allergic conjunctivitis treatment drug and the blepharoconjunctivitis treatment drug may include at least one selected from the group consisting of olopatadine, ketotifen, alcaftadine, bepotastine, azelastine, chlorpheniramine, neostigmine, pyridoxine, tetrahydrozoline, epinastine, naphazoline, chondroitin, panthenol, glycyrrhizic acid, aminocaproic acid, retinol, vitamin E, pheniramine, allantoin, chlorobutanol, taurine, aspartic acid, cyanocobalamin, enoxolone, benzalkonium chloride, azulene, acitazanolast, cromolyn, tranilast, pemirolast, N-acetyl aspartyl, lodoxamide, and N-acetyl aspartyl glutamic acid. However, the scope of the present disclosure is not limited thereto.

In an embodiment, one or more of the night blindness treatment drug and the amblyopia treatment drug may include at least one selected from the group consisting of bilberry dry extract, vitamin E, retinol, beta-carotene, ascorbic acid, pyridoxine, citrulline, tocopherol, riboflavin, fursultiamine, manganese, selenium, ergocalciferol, and cefaclor. However, the scope of the present disclosure is not limited thereto.

In an embodiment, the eye inflammation treatment drug may include at least one selected from the group consisting of fluorometholone, dexamethasone, tetryzoline, prednisolone, loteprednol, rimexolone, triamcinolone, hypromellose, naphazoline, chlorphenamine, bromfenac, ketorolac, bendazac, diclofenac, pranoprofen, flubiprofen, tobramycin, neomycin, polymyxin B, gentamicin, fluorometholone, chloramphenicol, and sulfamethoxazole. However, the scope of the present disclosure is not limited thereto.

In an embodiment, the cataract treatment drug may include at least one selected from the group consisting of potassium iodide, sodium iodide, pirenoxine, thiamine, azapentacene, bendazac lysine, prednisolone, nepafenac, and diclofenac. However, the scope of the present disclosure is not limited thereto.

In an embodiment, the antiviral agent may include at least one selected from the group consisting of acyclovir, ganciclovir, and trifluridine. However, the scope of the present disclosure is not limited thereto.

In an embodiment, the mydriatic drug may include at least one selected from the group consisting of tropicamide, phenylephrine, aminocaproic acid, atropine, cyclopentolate, and homatropine. However, the scope of the present disclosure is not limited thereto.

In an embodiment, the carbonic anhydrase inhibitor may include at least one selected from the group consisting of acetazolamide, methazolamide, dorzolamide, and brinzolamide. However, the scope of the present disclosure is not limited thereto.

In an embodiment, the macular degeneration treatment drug may include at least one selected from the group consisting of ranibizumab, bevacizumab, and verteporfin. However, the scope of the present disclosure is not limited thereto.

As described above, the contact lens 11 of the present embodiment may include a plurality of drug chambers 122, and the plurality of drug chambers 122 may contain different drugs D such as the above-listed drugs. In another embodiment, the plurality of drug chambers 122 may contain the same drug D.

In the related art, a composition, in which a drug to be administered and a polymer for controlling the release of the drug are mixed with each other, is applied to the surface of a lens surface or filled in a drug storage structure. According to the related art, however, the polymer is provided to the eyes of a user together with the drug, causing a foreign body sensation in the eyes. However, according to the present embodiment, a drug is provided to the eyes of a user through the cover member, thereby preventing a foreign body sensation which occurs in the related art.

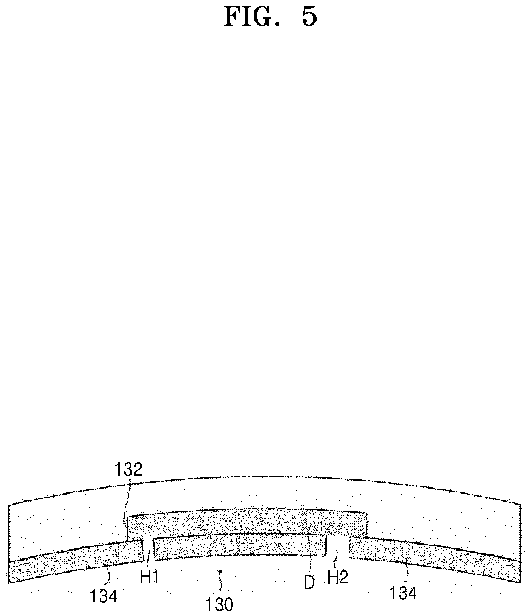

Hereinafter, another implementation example of a contact lens according to the second embodiment will be described with reference to FIG. 5. However, descriptions of elements, which are the same as or similar to the elements described above, may be omitted. FIG. 5 is a cross-sectional view schematically illustrating another implementation example of a contact lens according to the second embodiment. Referring to FIG. 5, the contact lens of the present embodiment includes a drug storage structure 130. The drug storage structure 130 includes: a drug chamber 132 having a concave structure in the contact lens to store a drug in the concave structure; a cover member 134 covering at least the drug chamber 132; and a discharge structure formed on the cover member 134 to provide the drug to the eyeball.

In the illustrated implementation example, the discharge structure includes one or more holes H1 and H2. The one or more holes H1 and H2 may have the same cross-sectional area or different cross-sectional areas. In an implementation example which is not shown, the discharge structure includes one hole. In the implementation example illustrated in FIG. 5, each of the holes H1 and H2 has the same cross-sectional area at a side of the drug storage chamber 132 and a side of the eyeball of a user. However, in an implementation example which is not shown, one of the holes H1 and H2 may have different cross-sectional areas at the side of the drug storage chamber 132 and the side of the eyeball of a user.

In addition, the cross-sectional area of the one or more holes H1 and H2 may be adjusted such that the drug stored in the drug chamber 132 may be discharged by capillary action, and thus the drug may be easily supplied from the drug chamber 132 to the user's eyeball. Furthermore, the hourly dose of the drug stored in the drug chamber 132 may be adjusted by adjusting the cross-sectional area of the holes H1 and H2.

In the implementation example, the cover member 134 may include a silicone material. In another example, the cover member 134 may a microporous polymer to provide the drug to the user through the cover member 134 as well as the holes of the discharge structure. The cover member 134 may include at least one selected from the group consisting of: the same polymer as that included in the lens; the above-mentioned water-soluble polymer; and the above-mentioned water-insoluble polymer.

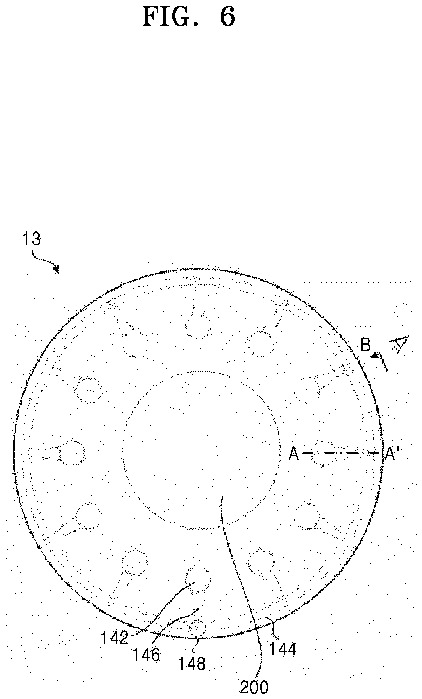

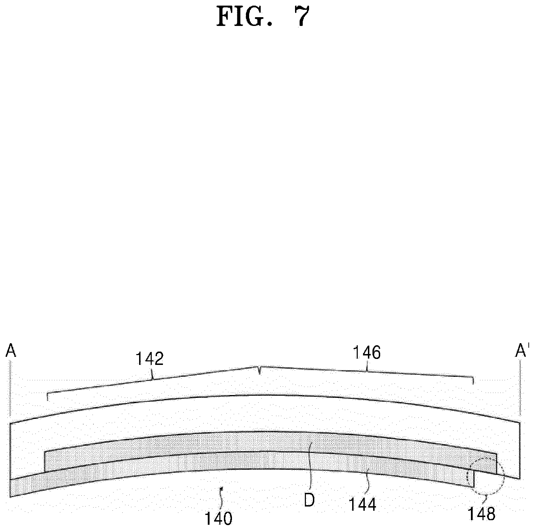

Hereinafter, another implementation example of a contact lens according to the second embodiment will be described with reference to FIGS. 6 and 7. However, descriptions of elements, which are the same as or similar to the elements described above, may be omitted. FIG. 6 is a plan view schematically illustrating another implementation example of a contact lens according to the second embodiment. FIG. 7 is a schematic cross-sectional view taken along line A-A' of FIG. 6. Referring to FIGS. 6 and 7, a contact lens 13 includes a drug storage structure 140. The drug storage structure 140 includes: a drug chamber 142 having a concave structure to store a drug in the concave structure; a drug discharge path 146 having a concave structure and connected to the drug chamber 142 to release the drug therethrough; and a cover member 144 covering the drug discharge path 146 and the drug chamber 142, where the cover member 144 does not cover an end portion of the drug discharge path, thereby forming a drug outlet 148.

The drug discharge path 146 is connected to the drug chamber 142 to guide the drug D contained in the drug chamber 142 to the drug outlet 148. FIG. 7 shows an example in which the drug discharge path 146 and the drug chamber 142 are formed at the same height. However, in an example which is not shown, the height of the drug discharge path 146 may be less than the height of the drug chamber 142.

The cover member 144 may be formed to cover a rear surface of the contact lens 13 as illustrated in FIG. 6, and may not cover a lower end portion of the drug discharge path 146 to form the drug outlet 148.

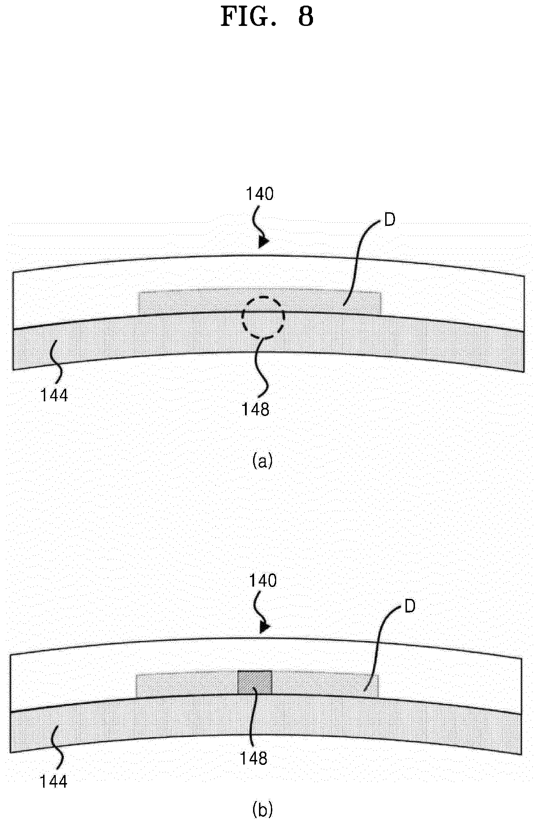

FIG. 8A is a cross-sectional view of the contact lens, which illustrates an example of the drug outlet 148 in the direction B in FIG. 6, and FIG. 8B is a view illustrating another example of the drug outlet 148. Referring to FIGS. 6 to 8A, the cover member 144 may not cover the lower end portion of the drug discharge path 146 to form the drug outlet 148. Therefore, according to the example illustrated in FIG. 8A, the drug outlet 148 discharges the drug D to a lower surface of the contact lens 13.

In the example of the drug outlet illustrated in FIG. 8B, however, the cover member 144 may entirely cover a lower surface of the drug discharge path 146 but may not cover a lateral end portion of the drug discharge path 146, such that the drug outlet 148 may be formed in an end portion of the drug discharge path 146 as shown in FIG. 8B.

People involuntarily blink their eyelids. A user of the contact lens 13 may blink the eyelid involuntarily, and thus the drug D stored in the drug chamber 142 may be discharged through the drug outlet 148 and provided to the eyeball of the user by the pressure created as the eyelid moves on an upper portion of the contact lens 13.

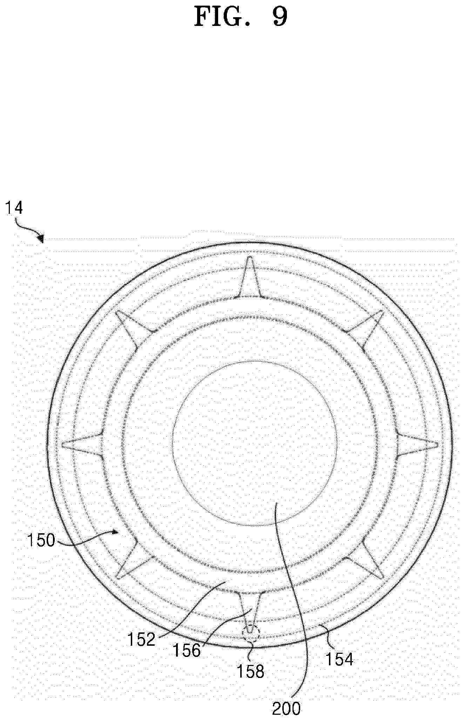

Hereinafter, another implementation example of a contact lens according to the second embodiment will be described with reference to FIG. 9. However, descriptions of elements, which are the same as or similar to the elements described above, may be omitted. FIG. 9 is a plan view schematically illustrating another implementation example of a contact lens according to the second embodiment. Referring to FIG. 9, a contact lens 14 includes a drug storage structure 150. The drug storage structure 150 includes: a drug chamber 152 having a concave structure to store a drug in the concave structure; a drug discharge path 156 is formed in a concave structure in the contact lens and connected to the drug chamber 152 to release the drug; and a cover member 154 covering the drug discharge path 156 and the drug chamber 152, wherein the cover member 154 does not cover an end portion of the drug discharge path to form a drug outlet 158.

According to the illustrated example, the drug chamber 152 may be formed in a circular shape around the center of the contact lens 14. Therefore, a drug may be stored in a large amount.

In the illustrated contact lens 14, the drug outlet 158 is formed on a lower surface of the contact lens 14 as in the example described above to discharge a drug D, and the drug outlet 158 may be formed on a side of the drug discharge path 156 to discharge the drug D to an end portion of the contact lens 14.

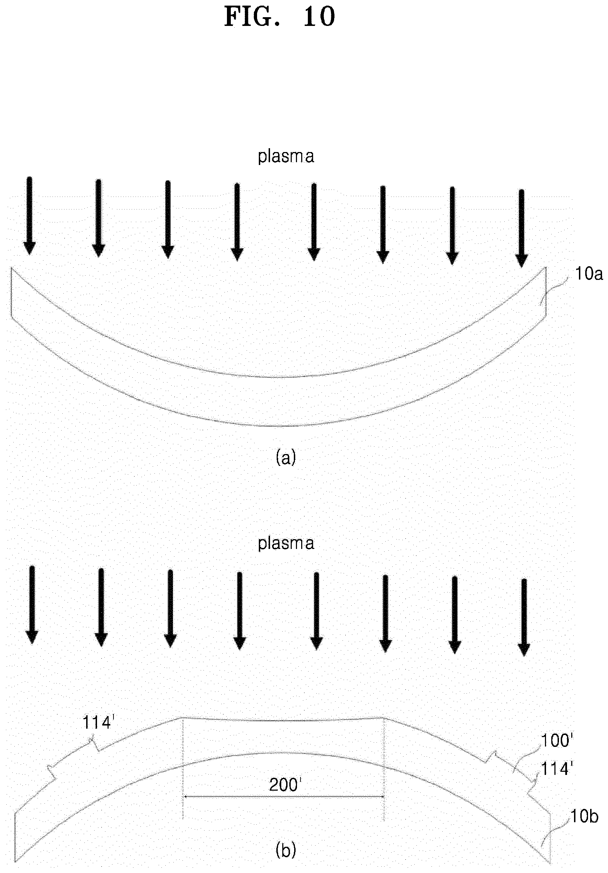

Hereinafter, a method of manufacturing a contact lens will be described according to an embodiment with reference to FIGS. 10A and 10B. FIGS. 10A and 10B are views illustrating a mold for manufacturing a contact lens according to the embodiment. FIG. 10A is a cross-sectional view schematically illustrating a first mold 10a, and FIG. 10B is a view schematically illustrating a second mold 10b. Referring to FIGS. 10A and 10B, the mold according to the present embodiment includes the first mold 10a and the second mold 10b.

When manufacturing a contact lens, a polymer is arranged on the first mold 10a, the second mold 10b is positioned on the polymer, and the polymer is cured to form a contact lens having a desired shape. For example, the process of curing the polymer may be performed by applying heat to the polymer.

In the embodiment illustrated in FIGS. 10A and 10B, the second mold 10b includes: a structure 100' for forming a concave structure of a contact lens; a structure 114' forming a gas discharge structure; and a structure 200' for forming a vision correction lens portion such that the contact lens has a desired diopter. However, in an embodiment not shown, the structure 100' and the structure 200' may be formed on the first mold 10a.

Furthermore, in an embodiment not shown, a structure for forming a concave structure may be formed on the second mold 10b, but a structure for forming a gas discharge structure may not be formed on the second mold 10b.

In the related art, molds for manufacturing contact lenses have a problem in that a contact lens material sticks to the molds while contact lenses are formed using the molds. To address this problem, contact lenses are manufactured using disposable molds in the related art.

In the present embodiment, however, as shown in FIGS. 10A and 10B, surfaces of the molds 10a and 10b, which are to be brought into contact with a polymer for forming a contact lens, is plasma treated using Teflon gas as shown in FIGS. 10A and 10B. The surfaces of the molds 10a and 10b are hydrophobically modified through the plasma treatment and are coated with Teflon to a thickness of several thousand angstroms (Å) by a plasma polymerization of C4F8, and thus a contact lens formed by curing a polymer is easily removed from the molds 10a and 10b. Therefore, compared to dispensable molds of the related art, the mold 10 may be semi-permanently used to reduce costs for mass production of lenses.

### Implementation examples and experimental examples

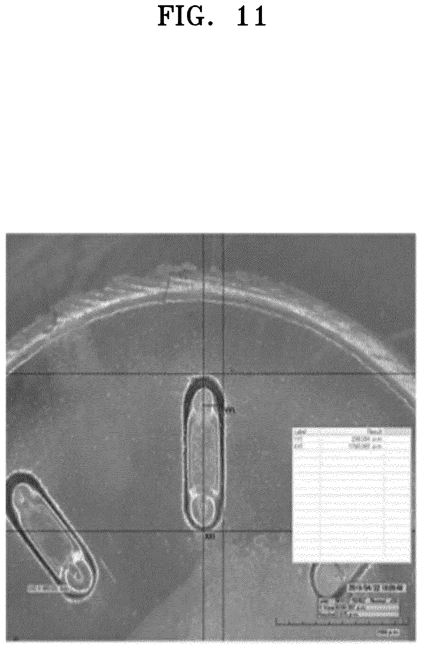

Hereinafter, implementation examples and experimental examples of contact lenses will be described according to embodiments with reference to FIGS. 11 to 13. A mold was made of 304-grade stainless steel, and machined into a three-dimensional shape using a milling machine (CNC machine 850), and surface polished after the machining to increase light transmittance. The radius of curvature of a mold 10b for forming structures for storing tears or a drug to be in contact with the eyeball and the radius of curvature of an external mold 10b are respectively 8.8 mm and 8.6 mm.

According to the implementation example, a contact lens was manufactured using a mixture in which 98% of 2-hydroxyethylmethacrylate (2-HEMA) was mixed with 2% of a crosslinking agent (EGDMA, ethylene glycol dimethacrylate) for increasing stability, an initiator (AIBN, 2,2'-azobis(2-methylpropionitrile)), and an organic compound (MAA, methacrylic acid) for increasing the moisture content of the contact lens.

FIG. 11 is an enlarged image taken by a three-dimensional digital microscope (Hl-ROX, KH-7700) to illustrate the structure of the contact lens of the implementation example and measure structural dimensions. It is seen that a tear storage structure 110 and gas discharge structures 114 were successfully formed, and the total volume is 1.4 mm³.

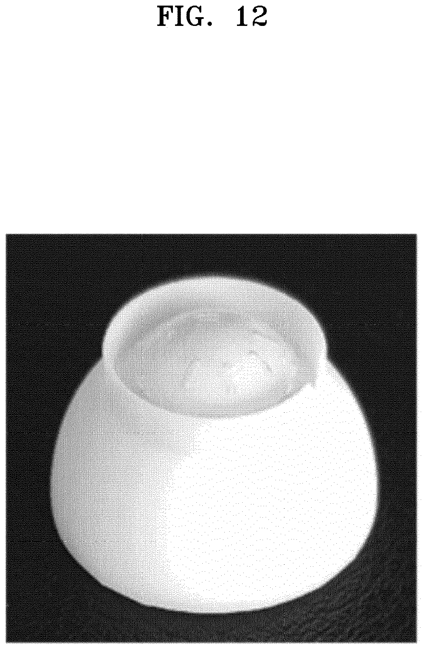

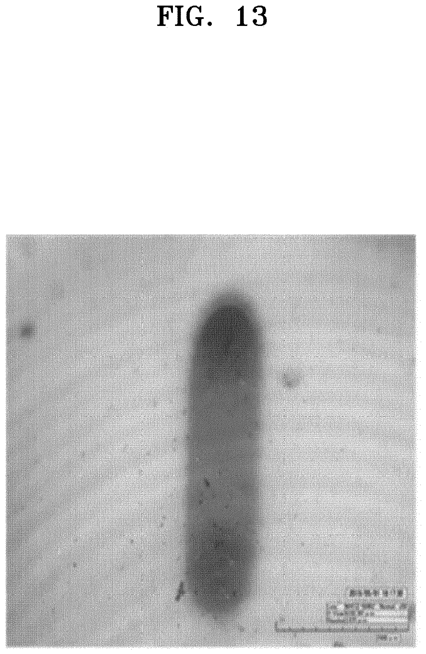

FIG. 12 is an image illustrating the manufactured lens mounted on the pupil of an artificial eyeball to test the tear storage effect of the manufactured lens. FIG. 13 is an image illustrating fluid stored in the tear storage structure as a result of a tear storage experiment performed using the artificial eyeball. An external syringe pump (KD scientific, KDS-100) was used to secrete the fluid through the lacrimal duct of the artificial eyeball. To visually grasp results of the experiment, a fluid in which water and blue ink was mixed with together was used as virtual tears. After injecting the fluid at a flow rate of 0.6 ml per minute for 20 seconds, water was further injected to remove ink on the outside of the tear storage structure and check again whether or not the tears were stored in the tear storage structure. All processes were observed with a three-dimensional digital microscope (HIROX, KH-7700). As a result of the experiment, it was confirmed that the fluid was stored inside the proposed lens structure.

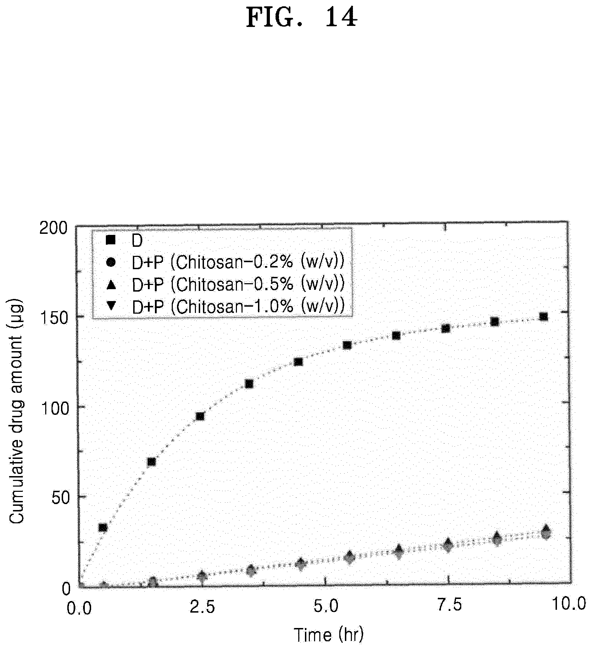

Hereinafter, results of a drug release experiment, which was performed on the contact lens illustrated in FIGS. 3 to 4 according to the embodiment, will be described with reference to FIGS. 14 to 15. Cyclosporine (Sigma-Aldrich, Munich, Germany) was used as a drug. The drug was prepared by weighing 160 mg of cyclosporine in 6 ml of 80% (v/v) ethanol. A cover member was formed of a microporous polymer. The porous polymer was prepared by adjusting the content of HPMC 100000 (hydroxypropyl methylcellulose 2208, 90SH-105, Shin-Etsu Chemical Co., Ltd., Tokyo, Japan) and the content of chitosan in 10 mL of purified water, and PEG 300 (BASF Ludwigshafen, Germany) was added as a plasticizer to 2 %(w/v).

A total of 25 µl of the prepared drug was injected into the drug storage structure of the contact lens by using a syringe and was dried at 45 °C for 1 hour, and 35 µl of the microporous polymer was applied thereto and dried at 45° C for 1 hour.

The drug release experiment was performed as follows. The contact lens was fixed between a glass plate and a Franz Diffusion Cell. A Franz Diffusion Cell System (Logan Instruments Corp, VTC 300, NJ) was stirred at 35°C using a stirrer.

Sample collection times were set to 0.5, 1.5, 2.5, 3.5, 4.5, 5.5, 6.5, 7.5, 8.5, and 9.5 hours when 0.05 M of pH 7.4 phosphate buffer solution was used as a buffer. The collected sample solution was filtered using a 0.45 µm membrane filter and was used as a sample for quantitative analysis.

Table 1 shows the compositions of the drug (D) and the cover member (P).

**[Table 1]**

| COMPONENTS | | CHITOSAN 0.2% | CHITOSAN 0.5% | CHITOSAN 1 % |
|---|---|---|---|---|
| D | CYCLOSPORINE | 400 µg | 400 µg | 400 µg |
| P | CHITOSAN | 70 µg | 175 µg | 350 µg |
| | PEG 300 | 700 µg | 700 µg | 700 µg |

FIG. 14 is a view illustrating drug release patterns for contents of chitosan. D = 25 µl of a drug layer (cyclosporine 16 mg/ml) was dispensed. P = 30 µl of a polymer layer (chitosan + PEG 2% (w/v)) was dispensed

In FIG. 14, a curve indicated with red circles overlaps a D+P (Chitosan - 0.5% (w/v) curve indicated with upward triangles and a D+P (Chitosan - 1.0% (w/v) indicated with downward triangles.

The cumulative drug release in the absence of the cover member was fitted by nonlinear curve-fitting as a first-order exponential function, and the rate of release was analyzed with a time constant _{T}. The time (time constant) necessary for reaching 63% of the maximum drug accumulation was measured to be 2.54 hours. When chitosan was used as the material of the cover member, the cumulative drug release was linearly curve-fitted as a linear function, and the release characteristics were evaluated with the slope α of the linear function.

Results of the experiment showed that when no cover member used, the drug release increased exponentially with time, and when chitosan was used as the cover member, the drug release did not seem to have a correlation with the content of chitosan and increased linearly with time.

Table 2 shows the compositions of the drug (D) and the cover member (P).

**[Table 2]**

| COMPONENTS | | H:C=9:1 | H:C=7:3 | H:C=5:5 | H:C=3:7 | H:C=1:9 |
|---|---|---|---|---|---|---|
| D | CYCLOSPORINE | 400 µg | 400 µg | 400 µg | 400 µg | 400 µg |
| P | HPMC 100000 (H) | 315 µg | 245 µg | 175 µg | 105 µg | 35 µg |
| | CHITOSAN (C) | 7 µg | 21 µg | 35 µg | 49 µg | 63 µg |
| | PEG 300 | 700 µg | 700 µg | 700 µg | 700 µg | 700 µg |

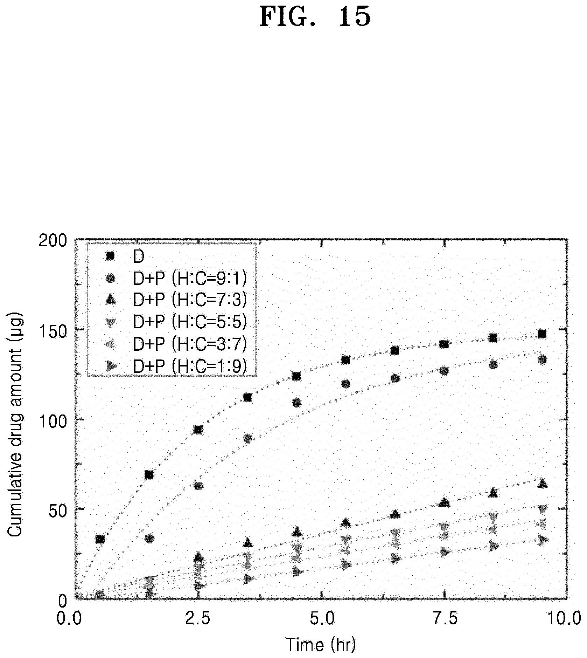

FIG. 15 is a view illustrating drug release patterns in the experiment for ratios of HPMC 100000 1 % + chitosan 0.2 %. D = 25 µl of a drug layer (cyclosporine 16 mg/ml) was dispensed. P = 30 µl of a polymer layer (HPMC 100000 1 % (w/v)): C (chitosan 0.2 % (w/v)) + PEG 2 % (w/v)) was dispensed. (H : C) = H (HPMC 100000 1 % (w/v)) : C (chitosan 0.2 % (w/v))

Results of the experiment showed that the cumulative drug release in the absence of the cover member increased exponentially with time. Although the drug release also increased exponentially when HPMC 100000 and chitosan were mixed at a ratio of 9: 1 and used as the cover member, as the amount of chitosan was increased, the drug release amount was changed in a linearly increasing pattern, and the cumulative drug release amount gradually decreased.

Therefore, through the experiments, it could be understood that the release of a drug filled in a contact lens may be controlled using a cover member made of a microporous polymer. In addition, the drug release time may be controlled by adjusting the types and contents of polymers of the cover member to maximally maintaining the efficacy of the drug.

As described above, various embodiments of the present disclosure have been described in detail, and those of ordinary skill in the art to which the present disclosure pertains may make various modifications therein without departing from the spirit and scope of the present disclosure as defined by the appended claims. Therefore, such modifications should be construed as being included within the scope of the present disclosure.

## Claims

1. A contact lens comprising:
a vision correction lens portion positioned in a center portion of the contact lens to refract light; and
a plurality of tear storage structures radially arranged from the center portion of the contact lens to store tears,
wherein each of the plurality of tear storage structures comprises a concave structure formed in the contact lens and having a first depth to store introduced tears.

2. The contact lens of claim 1, wherein the contact lens comprises a microporous polymer having micropores.

3. The contact lens of claim 1, wherein each of the plurality of tear storage structures further comprises a gas discharge structure which has a second depth greater than the first depth and is configured to discharge air remaining in the concave structure outward from the contact lens.

4. The contact lens of claim 1, wherein the plurality of tear storage structures comprise eight tear storage structures radially arranged from the center portion of the contact lens.

5. The contact lens of claim 1, wherein the plurality of tear storage structures store a total of 1.5 mm³ or less of tears.

6. A contact lens comprising:
a vision correction lens portion positioned in a center portion of the contact lens to refract light; and
a drug storage structure arranged apart from the center portion and configured to store a drug,
wherein the drug is provided to an eye of a user wearing the contact lens.

7. The contact lens of claim 6, wherein the contact lens comprises a microporous polymer having micropores.

8. The contact lens of claim 6, wherein the drug storage structure comprises:
a drug chamber having a concave structure and formed in the contact lens to store the drug in the concave structure; and
a cover member covering at least the drug chamber,
wherein the cover member comprises a microporous polymer in which micropores are formed, and
the drug is provided to the eye through the micropores.

9. The contact lens of claim 6, wherein the drug storage structure comprises:
a drug chamber having a concave structure and formed in the contact lens to store the drug in the concave structure;
a cover member covering at least the drug chamber; and
a discharge structure formed in the cover member to provide the drug to the eye.

10. The contact lens of claim 9, wherein
the discharge structure comprises one or more holes, and
the one or more holes have equal or different areas.

11. The contact lens of claim 6, wherein the drug storage structure comprises:
a drug chamber having a concave structure and formed in the contact lens to store the drug in the concave structure;
a drug discharge path having a concave structure and formed in the contact lens, the drug discharge path being connected to the drug chamber to release the drug; and
a cover member covering the drug discharge path and the drug chamber,
wherein the cover member does not cover an end portion of the drug discharge path, thereby forming a drug outlet.

12. The contact lens of claim 11, wherein the drug storage structure comprises:
a plurality of drug chambers which are apart from each other and not connected to each other; and
a plurality of drug discharge paths respectively connected to the plurality of drug chambers,
wherein the plurality of drug chambers store identical or different drugs.

13. The contact lens of claim 11, wherein the drug storage structure comprises:
a single drug chamber having a circular shape and formed in the contact lens; and
a plurality of drug discharge paths connected to the single drug chamber,
wherein the plurality of drug discharge paths discharge the drug to an end portion of the contact lens.

14. The contact lens of claim 6, wherein the drug comprises at least one selected from the group consisting of a dry eye syndrome treatment drug, a glaucoma treatment drug, an intraocular pressure lowering agent, an eye damage treatment drug, an antibacterial agent, an allergic conjunctivitis treatment drug, a blepharoconjunctivitis treatment drug, a night blindness treatment drug, an amblyopia treatment drug, an eye inflammation treatment drug, a cataract treatment drug, an antiviral agent, a mydriatic drug, a carbonic anhydrase inhibitor, and a macular degeneration treatment drug.

15. The contact lens of claim 8, wherein the microporous polymer comprises one selected from the group consisting of a water-soluble polymer and a water-insoluble polymer.

16. The contact lens of claim 15, wherein the water-soluble polymer comprises at least one selected from the group consisting of acacia, agar, alginic acid, carbomer, carrageenan, cellulose acetate, ceratonia, chitosan, chondroitin sulfate, dermatan sulfate, dextran, ethyl cellulose, gelatin, guar gum, hydroxyethyl cellulose, hydropropyl betadex, hydroxypropyl cellulose, hypromellose, hypromellose acetate succinate, hypromellose phthalate, karaya gum, locust bean gum, methylcellulose, molasses, pectin, polyacrylamide, polycaprolactone, polyethylene oxide, polyethylene glycol, polyhydroxyethylmethacrylate, polyorthoester, polyvinyl alcohol, polyvinylpyrrolidone, sodium alginate, carboxymethylcellulose, sodium hyaluronate, tragacanth, triethyl citrate, and xanthan gum.

17. The contact lens of claim 15, wherein the water-insoluble polymer comprises at least one selected from the group consisting of acetyl alcohol, acetyl ester wax, acetyl tributyl citrate, aluminum monostearate, carnauba wax, cellulose acetate, cellulose acetate phthalate, dibutyl sebacate, ethyl cellulose, glycerin monostearate, glyceryl behenate, glyceryl monooleate, glyceryl palmitostearate, hydrogenated castor oil, hydrogenated vegetable oil type 1, isopropyl palmitate, polycaprolactone, polyglycolide, polylactic acid, polylactide, polymethacrylate, polyoxyglyceride, shellac, stearic acid, stearyl alcohol, tributyl citrate, white wax, yellow wax, and zein.

18. A mold for forming a contact lens, the mold comprising:
a first mold; and
a second mold configured to cure a liquid porous polymer such that the contact lens has a concave structure with respect to the first mold,
wherein surfaces of the first mold and the second mold, which are to come into contact with the liquid porous polymer, are coated with Teflon.

19. The mold of claim 18, wherein the mold comprises stainless steel.

20. The mold of claim 18, wherein a surface of the mold is hydrophobically treated by C4F8 plasma polymerization treatment.
